# EUROPEAN PATENT SPECIFICATION

(11) **EP 1 888 045 B1**
(45) Date of publication and mention of the grant of the patent: **08.09.2010**
(21) Application number: 06831741.1
(22) Date of filing: 24.05.2006
(51) Int. Cl.: A61K 9/50, A61K 45/06

(54) **ORAL PHARMACEUTICAL COMPOSITION FOR TREATING A COX-2 MEDIATED CONDITION**
ORALE PHARMAZEUTISCHE ZUSAMMENSETZUNG ZUR BEHANDLUNG EINER COX-2-VERMITTELTEN ERKRANKUNG
COMPOSITION PHARMACEUTIQUE POUR LE TRAITEMENT DE MALADIES TRANSMISES PAR COX-2

(30) Priority: 24.05.2005 EP 05300407; 24.05.2005 US 683776 P; 24.05.2005 US 683777 P; 24.05.2005 EP 05300406
(43) Date of publication of application: 20.02.2008
(73) Proprietor: FLAMEL TECHNOLOGIES, 69200 Venissieux (FR)
(72) Inventor: SOULA, Gerard, 69330 Meyzieu (FR); GUIMBERTEAU, Florence, 33450 Montussan (FR)
(74) Representative: Fleurance, Raphaël
(86) International application number: PCT/IB2006/003659
(87) International publication number: WO 2007/036809

(56) References cited:
- EP-A- 0 103 991
- EP-A- 0 239 361
- EP-A- 0 411 590
- WO-A-02/066002
- WO-A-03/057166
- US-A- 5 603 957
- US-A1- 2002 051 814
- BECKER JAN C ET AL: "Current approaches to prevent NSAID-induced gastropathy - COX selectivity and beyond" BRITISH JOURNAL OF CLINICAL PHARMACOLOGY, vol. 58, no. 6, December 2004 (2004-12), pages 587-600, XP002349932 ISSN: 0306-5251
- SILVERSTEIN FRED E ET AL: "Gastrointestinal toxicity with celecoxib vs nonsteroidal anti-inflammatory drugs for osteoarthritis and rheumatoid arthritis: The CLASS study: A randomized controlled trial" JAMA (JOURNAL OF THE AMERICAN MEDICAL ASSOCIATION), vol. 284, no. 10, September 2000 (2000-09) , pages 1247-1255, ISSN: 0098-7484

## Description

### BACKGROUND OF THE INVENTION

In patients with established cardiovascular disease, aspirin use has been documented to decrease the risk of a primary myocardial infarction, stroke and vascular death. Aspirin may also be used to prevent cardiovascular events in patients with established cardiovascular disease such as a myocardial infarction, stroke, or angina. Generally, the use of aspirin in these individuals is recommended based on a documented decrease in future cardiovascular events and mortality.

Aspirin, or acetylsalicylic acid, acts to prevent platelet aggregation by irreversibly inhibiting cyclooxygenase (COX). There are many types of COX including COX-1, COX-2, COX-3 and COX-derived proteins, collectively known as COX. COX converts arachidonic acid to thromboxane, a potent vasoconstrictor and a platelet aggregation stimulator. Aspirin inhibits COX by acetylating it. The inhibition of COX activity by aspirin is generally irreversible. This is an important distinction for aspirin because the duration of the effects of aspirin is related to the turnover of COX in different tissue targets. Platelets are especially susceptible to aspirin mediated irreversible inactivation of COX because platelets have little or no capacity for protein biosynthesis and, thus, cannot regenerate the COX enzyme. In practical terms, this means that a single dose of aspirin will inhibit the platelet COX for the life of the platelet, 8-11 days.

When aspirin is absorbed from the digestive tract, it is collected by the portal vein. The portal vein then goes to the liver where the aspirin is deacetylated. Once deacetylated, aspirin no longer has the ability to acetylate COX. However, the mechanism in the liver can rapidly reach saturation causing the aspirin overflow to enter the systemic blood circulation. In the systemic circulation, aspirin that has not been deacetylated by the liver can further inhibit COX in other tissues and cells. For instance, in the endothelial cells that line the vasculature and the gastric endothelium, aspirin-induced COX inhibition results in a decrease of prostacyclin, which, contrary to thromboxane, is a potent vasodilator, a platelet aggregation inhibitor and a cytoprotector. Therefore, aspirin that enters the systemic blood circulation results in inhibition of the prostacyclin and other prostaglandins and which induces gastric side effects. This phenomenon of blind inhibition of the different prostaglandins in the organism is commonly referred to as the dilemma of aspirin. It is well known to scientists and has been widely described in the literature.

While aspirin is a very useful medication for the prevention of cardiovascular thrombotic events in patients with or those at risk for cardiovascular disease, there are serious side effects of aspirin administration. For example, the most common side effect is a propensity to induce gastric or intestinal ulceration, which may result in hemorrhaging. This effect occurs when acetylated aspirin inhibits COX in the systemic circulation. The COX in the systemic circulation catalyzes the biosynthesis of gastric prostaglandins that ordinarily serve as cytoprotective mucous in the intestines. As the gastric mucosa is no longer protected by these gastric prostaglandins, the gastric acid induces tissue damage and bleeding. The gastrointestinal effects of aspirin may be caused by its lack of selectivity between antiplatelet COX-1 inhibition and endothelial COX inhibition leading to gastric mucosal effects. The risk for gastrointestinal injury is observed in patients being treated with aspirin at dosages as low as 81 mg/day for cardioprotection.

Moreover, aspirin that is not metabolized by the liver may induce serious side effects. Since non steroidal anti inflammatories like aspirin may be taken by the patient for a substantial portion of his life, it is important to improve the safety profile of the treatment for the tens of millions of patients today who regularly take the drugs. Thus, it appears that there is a need in a therapeutic solution for treating the pathologies linked to platelet aggregation, or cardiovascular disease, without causing the serious side effects to the patients.

In patients with established cardiovascular disease, aspirin may have further side effects. For example, aspirin in the systemic circulation may decrease renal blood flow and the rate of glomerular filtration in patients with congestive heart failure. Therefore, acute renal failure may be precipitated. Aspirin may also promote the retention of salt and water by reducing the prostaglandin induced inhibition of both the reabsorption of chloride and the action of anti-diuretic hormone. This may cause edema in some patients who are treated with aspirin and may reduce the effectiveness of anti-hypertensive regimens. Aspirin and other COX inhibitors may also increase the risk of heart disease. Metabolism of arachidonic acid by COX results in the production of prostaglandins, which promotes inflammation. Therefore inhibition of COX results in decreased inflammation. However, COX inhibition also promotes arachidonic acid to be converted to pro-inflammatory agents such as leukotriene B4 and thromboxane A2. The resulting increase in these pro-inflammatory agents may lead to increased atherosclerosis and platelet aggregation, and other complications such as stroke and heart attack.

There therefore exists a need for a formulation which inhibits platelet aggregation and limits cardiovascular risks. Cardiovascular diseases of particular concern are the diseases resulting from excessive and uncontrolled platelet aggregations (platelet disorders). Diseases caused by an excess of thromboxane often are treated with antithrombotics. It is, however, greatly desired to enhance the safety of these antithrombotic treatments. Notably, the thrombotic cardiovascular events of concern are those such as stroke, myocardial ischemia, myocardial infarction, angina pectoris, transient ischemic attack, reversible ischemic neurological deficits, and any similar thrombotic event in any vascular bed (splanchnic, renal, aortic, peripheral, etc.).

Further, chronic COX mediated diseases such as inflammatory diseases, rheumatoid arthritis and systemic lupus erythematosis are often treated with Non Steroidal Anti Inflammatory Drugs (NSAIDs) such as aspirin. A large number of patients, who are treated with common NSAIDs, or with more specific NSAIDs such as COX-2 inhibitors, have severe side effects. These severe side effects may include life threatening ulcers and thrombotic cardiovascular events that limit the therapeutic potential of said NSAIDs.

In addition, there is evidence that patients with chronic inflammatory conditions are at increased risk for thrombotic cardiovascular events. Furthermore, many patients treated with NSAIDs or suffering from chronic COX-2 mediated disease or condition are elderly and thus are at increased risk for thrombotic cardiovascular events. Thus, it is desirable to treat such patients with appropriate antiplatelet therapy, such as low dose aspirin.

Thus, it appears that there is a need in a therapeutic solution for treating the inflammatory disorders and the associated pathologies linked to platelet aggregation; without posing serious cardiovascular risks to the patients.

It has been previously proposed to combine low dose aspirin with COX-2 inhibitors to make an anti-inflammatory therapy, while decreasing the risk of a thrombotic cardiovascular event. WO 03/057 166 discloses pharmaceutical compositions comprising enteric-coated aspirin and COX-2 selective inhibitor.

Several methods have been tried for decreasing the risk of thrombotic cardiovascular events associated with inflammatory disorder treatment. For instance, it is known to administrate to the patients two major anti-platelets drugs, namely low dose aspirin and clopidogrel, to inhibit platelet aggregation and limit the inherent cardiovascular risks. Clopidogrel bisulfate is an inhibitor of platelet aggregation acting by direct inhibition of adenosine diphosphate binding to its receptor and of the subsequent activation of the glycoprotein GPIIb/ IIIa complex. Chemically, it is methyl (+)-( S)-a -(2- chlorophenyl)-6,7-dihydrothieno[3,2-c] pyridine-5(4H)-acetate sulfate (1:1). There are, however, considerable side effects of the treatment such as gastrointestinal hæmorrhage; neutropenia, agranulocytosis, gastrointestinal events such as abdominal pain, dyspepsia, gastritis and constipation, peptic, gastric or duodenal ulcers, diarrhea, rash and other skin disorders.

Still others suggest replacing low dose aspirin with nitric oxide releasing aspirin. For instance, WO-A-03/094924 discloses a method for treating a chronic COX-2 mediated disease or condition and reducing the risk of a thrombotic cardiovascular event. To a patient at risk of a thrombotic cardiovascular event a COX-2 selective inhibitor and nitric oxide releasing aspirin are orally administered concomitantly or sequentially,. The amount administered is sufficient to reduce the risk of thrombotic cardiovascular events while maintaining a high level of upper gastrointestinal safety and tolerability. WO-A- 03/033001 further proposes using aspirin in lower dosages than those previously implemented, namely 75 - 325mg per day. However the immediate release dose of aspirin included in that composition may still create many of the gastric side effects previously mentioned.

In addition, US-B-6,599,529 discloses an oral pharmaceutical modified-release multiple-units composition for the administration of a therapeutically and/or prophylactically effective amount of a NSAID substance, for instance aspirin, to obtain both a relatively quick onset of the therapeutic effect and to maintain a therapeutically active plasma concentration for a relatively long period of time. The modified release multiple-units composition has at least a first and a second fraction of multiple units. The first fraction is an immediate release form of NSAIDs, which comprises individual units that are designed to quickly release the drug substance. The second fraction is a delayed/sustained release form, which comprises individual units that are designed to slowly release the drug substance to enable a delayed and extended release of the drug substance. Typically, the second fraction comprises multiple units which are coated with a sustained release coating designed to release the drug substance in such a manner that the maintenance of a therapeutically active plasma concentration for a relatively long period of time are obtained. It may be administered once or twice a day. The pellet core is polysorbate 20, cellulose microcrystalline, lactose, carmellose sodium, maltodextrin and pregelatinized starch. Further, the inner coat contains hypromellose (Methocel E prem), magnesium stearate, talc, Eudragit NE 30 D. The outer coat contains hypromellose (Methocel E5 prem) and talc. The composition can comprise a further active drug substance selected from the group consisting of an antidepressant, an opioid, a prostaglandin analog, a glucocorticosteroid, a cytostaticum, a H2 receptor antagonist, a proton pump inhibitor and an antacid. Unfortunately, the immediate release fraction of NSAIDs, i.e., aspirin, in this composition still creates all of the gastric side effects mentioned before. The aspirin dilemma is therefore not solved.

Further, US-A-2004/0121004 and US-A-2004/0131676 disclose a non-enterically coated dosage form comprising: a proton pump inhibitor such as lansoprazole, a buffer, and a NSAID such as 50 - 100 mg of aspirin. The applications further disclose a method of treating conditions such as angina, aorto-pulmonary shunt occlusion, colorectal cancer, esophageal cancer, colon cancer, coronary artery disease, dementia, dysmenorrhea, myocardial infarction, rheumatoid arthritis, osteoarthritis, pain, headache, migraine headache, stroke, thrombocythemia, post-operative thromboembolism, ischemia, bursitis, cognitive decline, fever, gout, musculoskeletal disorders, soft tissue injury, and pericarditis. The method comprises administering to a patient having one or more of the above conditions a non-enterically coated dosage form. This form is an immediate release form of acetylsalicylic acid at alkaline pH. Granulates of NSAIDs are prepared from magnesium hydroxide, buffer, calcium carbonate, mannitol, avicel (micro-crystalline cellulose), and PVPP (cross-povidone). These granulates are tabletted. The immediate release of NSAIDs, i.e., aspirin, in this composition is still creating all gastric side effects mentioned before. The aspirin dilemma is not solved.

Still others propose treating the gastric bleeding and petechia side effects by coadministering large amounts of proton pump inhibitor to increase the gastric pH and reduce the pain. The drawbacks of this approach are that the damage to the gastric mucosa is very high and permanent and the large dose of proton pump inhibitor results in a constant high pH, which is deleterious for chronic use.

Moreover, US-B-5,603,957, which belongs to the applicant discloses and claims a pharmaceutical form comprising microcapsules for the controlled release of acetylsalicylic acid where the microcapsules consist of particles of acetylsalicylic acid with a size of between 100 and 1000 µm. These microcapsules are coated and designed so that, when ingested orally in a single administration of a dose of between 50 and 325 mg of acetylsalicylic acid, they induce moderate acetylsalicylic acid absorption kinetics in vivo in man. The absorption extends over at least 24 hours, the acetylsalicylic acid absorption being less than or equal to 10% by weight of the absorbed fraction of the dose at a time t after ingestion of 0.4 hour, less than or equal to 50% by weight of the absorbed fraction of the dose at t=3.9 hours, and less than or equal to 90% by weight of the absorbed fraction of the dose at t=23 hours, t being given to within +/-10%. It is also possible to create microcapsules of even smaller size, such as 50µm or less. One method to create such smaller microcapsules is disclosed in USPN 6,022,562 to Autant et al., which is owned by the Applicant.

The inventors have surprisingly found that they can use the pharmaceutical composition of microcapsules to selectively inhibit the COX in the portal vein and/or in the liver to reduce the production of thromboxane. Further, the pharmaceutical composition minimizes cyclooxygenase COX inhibition in the systemic circulation to optimize the inhibition of platelet aggregation. This subsequently prevents and/or treats cardiovascular diseases and risks associated with anti-inflammatory drugs used in the treatment of chronic COX mediated diseases or conditions, while minimizing the side effects.

### SUMMARY OF THE INVENTION

The oral pharmaceutical formulation of the invention in defined in the claims.

The inventors have surprisingly found that they can use the pharmaceutical composition of acetylsalicylic acid-based microcapsules to selectively inhibit the COX in the portal vein and/or in the liver to reduce the production of thromboxane. Further, the pharmaceutical composition minimizes COX inhibition in the systemic circulation to optimize the inhibition of platelet aggregation. This subsequently prevents and/or treats cardiovascular diseases and risks associated with anti-inflammatory drugs used in the treatment of chronic COX-mediated diseases or conditions, while minimizing the side effects.

The inventors have discovered novel oral pharmaceutical compositions for the prevention and/or the treatment of cardiovascular and inflammatory diseases. Cardiovascular diseases of particular concern for the invention are the diseases resulting from excessive and uncontrolled platelet aggregations (platelet disorders). More particularly, these diseases are those caused by an excess of thromboxane and against which antithrombotic treatments can be proposed to the patients.

The oral pharmaceutical composition of the instant invention may also be used for the prevention and/or the treatment of chronic COX-mediated diseases or conditions, i.e., the inflammatory diseases or conditions, while reducing the risk of thrombotic cardiovascular events.

Certain embodiments also address methods of prevention and/or treatment of these diseases, using these oral compositions. For instance, certain embodiments of the instant invention include administration of the oral pharmaceutical composition while enhancing the safety of antithrombotic treatments. In the present exposure, the "thrombotic" troubles denote, notably, the thrombotic cardiovascular events such as such as stroke, myocardial ischemia, myocardial infarction, angina pectoris, transient ischemic attack, reversible ischemic neurological deficits, and any similar thrombotic event in any vascular bed (splanchnic, renal, aortic, peripheral, etc.).

Certain embodiments contemplate oral pharmaceutical compositions that combine acetylsalicylic acid with anti-platelet aggregation drugs, without inducing gastric side effects.

### SUMMARY OF FIGURES

FIG. 1 depicts a graph showing the *in vitro* release profiles for the controlled release-acetylasalicylic acid-based microcapsules prepared according to Example 1 in accordance with a preferred embodiment of the present invention.
FIG. 2 depicts a graph showing the *in vitro* release profiles for the controlled release-omeprazole based microcapsules prepared according to Example 2 in accordance with a preferred embodiment of the present invention.

### DETAILED DESCRIPTION

The inventors have suprisingly found that they can use the pharmaceutical composition of acetylsalicylic acid-based microcapsules to selectively inhibit the COX-1 in the portal vein and/or in the liver to reduce the production of thromboxane. Further, the pharmaceutical composition minimizes COX inhibition in the systemic circulation to optimize the inhibition of platelet aggregation. This subsequently prevents and/or treats cardiovascular diseases and risks associated with anti-inflammatory drugs used in the treatment of chronic COX-mediated diseases or conditions while minimizing the side effects. Further, the inventors have discovered novel oral pharmaceutical formulations for the prevention and/or the treatment of cardiovascular and inflammatory diseases. Cardiovascular diseases of particular concern for the invention are the diseases resulting from excessive and uncontrolled platelet aggregations (platelet disorders). Mote particularly, these diseases are those caused by an excess of thromboxane and against which antithrombotic treatments can be proposed to the patients.

While not wishing to be constrained by any mode of action, the inventors believe that by coating the AcetylSalicylic Acid (ASA) to form microcapsules, they promote the direct action of aspirin on only the COX of the blood platelets in the hepatic portal circulation. The pharmaceutical formulation results in a low, constant release rate of acetylsalicylic acid from the microcapsules, and absorbed through the portal vein. This low release rate of acetylsalicylic acid is sufficient to inhibit COX in the portal system, and therefore prevent thromboxane formation and platelet aggregation. Once acetylsalicylic acid is deacetylated, it is no longer active in that it can no longer inhibit COX.

Faced with a significant output, the liver can quickly be saturated with acetylsalicylic acid, and any acetylsalicylic acid that is not deacetylated would overflow into the systemic blood stream.

But the inventors find that the use of said ASA microcapsules results in a release rate of acetylsalicylic acid low enough so that the gastrointestinal and hepatic first-pass metabolism is not saturated. Therefore, once the low release rate of acetylsalicylic acid inhibits the COX in the platelets in the portal system, any remaining active acetylsalicylic acid is then deacetylated in the liver. This results in minimal - if any - acetylsalicylic acid overflow into the systemic circulation to inhibit prostaglandin and prostacyclin production, thus preventing damage to the gastric endothelium and other side effects of ASA.

Optionally, the composition also contains a small amount of a gastric acid suppressing agent to completely suppress the gastric damage without significantly increasing the gastric pH. Preferably, said gastric acid suppressing agent is a proton pump inhibitor. Further, the amount of optional gastric acid suppressing agent used to increase the pH of the stomach would be just enough to decrease the dissolving of aspirin in the stomach. Further, the gastric acid suppressing agent may minimize the damage from any residual non-deacetylated acetylsalicylic acid coming from the liver and the portal blood circulation and entering the systemic blood circulation.

While not wishing to be constrained to low doses of gastric acid suppressing agent, the inventors find it surprising that one can greatly minimize gastric damage with only a minimal dose of a gastric acid suppressing agent. The low dose of the gastric acid suppressing agent in the present invention only slightly increases the gastric pH. Further, the low, constant dose of acetylsalicylic acid released in the intestinal tract would sufficiently inhibit COX in the portal circulation, while having minimal effects on the systemic prostaglandins. Therefore, the applicant takes credit for demonstrating that the combination of NSAIDs with a controlled release acetylsalicylic acid microcapsules and with at least one gastric acid suppressing agent makes it possible to increase the safety of the anti-platelet drug while decreasing the side effects.

The oral pharmaceutical composition as defined in the claims comprises a combination of at least one active principle and microcapsules for the controlled release of acetylsalicylic acid into the gastrointestinal environment. The microcapsules have an *in vitro* release profile, in 0.05M potassium dihydrogenophosphate/sodium hydroxide buffer medium pH 6.8, such that about 70% of the acetylsalicylic acid is released over a period of time of between about 2 and 20 hours. Further, the composition would inhibit COX notably in the portal vein to limit the production of thromboxane, while maintaining COX in the systemic blood stream. The composition, therefore, would limit the inhibition of the production of prostaglandin and prostacyclin to protect the gastric endothelium and to maintain its vasodilation properties.

The active principle is a drug used to treat a chronic COX-mediated disease or condition in the oral pharmaceutical formulation and is selected in the sub-class of the class of NSAIDs comprising the specific inhibitors of COX-2.

Generally, the COX inhibitor may be present between about 1 to 1000mg, preferably about 5 to about 500mg. For example, the recommended dosage for one particular COX inhibitor, celecoxib, is typically 100 mg twice per day or 200 mg once per day. Celecoxib is a preferred COX inhibitor in the compositions and methods of the present invention and may typically be present at 50-500 mg per unit dose. Especially preferred are methods and compositions utilizing 100 to 400 mg celecoxib. As another example, rofecoxib for oral administration used to be available in tablets of 12. 5, 25 or 50 mg and in an oral suspension containing either 12.5 mg or 25 mg rofecoxib per 5 ml, the recommended initial daily dosage for the management of acute pain being 50 mg. Peak plasma concentrations of rofecoxib typically occur about 2-3 hours after oral administration and the drug has a half life of about 17 hours.

Unless otherwise indicated, use of the term "about" in this invention description is intended to mean plus or minus 10% of the designated amount; thus, "about 5 to 80%" would mean a range of 4.5-5.5% to 76-84%.

In some embodiments, an oral pharmaceutical composition may comprise at least one immediate COX-2 inhibitor form and/or at least one controlled release COX-2 inhibitor form.

The term "controlled release" denotes, in the present disclosure, a prolonged or sustained release and/or a delayed release and/or a pulsed release of active principle by an oral pharmaceutical formulation. Such a controlled-release oral pharmaceutical formulation may, for example, comprise an immediate-release phase and a slow-release phase. Modified-release medicinal products are well known in this field; see, for example, Remington: The Science and practice of pharmacy", 19th edition, Mack Publishing Co. Pennsylvania, USA. The modified release may in particular be a prolonged and/or delayed release.

With respect to active principles and aspirin, it is expected that the skilled practitioner will adjust dosages on a case by case basis using methods well established in clinical medicine. The daily dosage may be provided in either a single or multiple regimen with the latter being generally referred. These are simply guidelines since the actual dose must be carefully selected and titrated by the attending physician based upon clinical factors unique to each patient. The optimal daily dose will be determined by methods known in the art and will be influenced by factors such as the age of the patient, the disease state, side effects associated with the particular agent being administered and other clinically relevant factors.

It is apparent from the foregoing text that the microcapsules of the invention should be very effective in pharmacological terms, perfectly tolerated by the organism, especially as regards gastric tolerance, capable of being presented in various appropriate pharmaceutical forms and easy and inexpensive to obtain. Further, the controlled release acetylsalicylic acid microcapsules have high selectivity for the thromboxane inhibition, which makes it possible to maintain the production of prostacyclin, in order to protect the gastro-intestinal tract. Furthermore, it is contemplated that the gastric acid suppressing agent maintains the pH of the stomach high enough to reduce the acidic erosion of the surface of the stomach and even to facilitate the healing process when some ulceration occurs.

The microcapsules can be orally ingestible and comprise particles of acetylsalicylic acid with a size of less than about 1000 µm, preferably between about 50 µm or 100 µm to 1000 µm. The microcapsules are coated and designed so that when ingested orally in a single administration, the microcapsules induce acetylsalicylic acid absorption kinetics *in vivo* in man, extending over at least 24 hours. The acetylsalicylic acid absorption is less than or equal to 10% by weight of the absorbed fraction of the dose at 0.4 hour post ingestion, less than or equal to 50% by weight of the absorbed fraction of the dose at 3.9 hours post ingestion, and less than or equal to 90% by weight of the absorbed fraction of the dose at 23 hours post ingestion.

In some preferred embodiments of the invention, the *in vivo* acetylsalicylic acid absorption kinetics takes place over a period such that the absorption would be less than or equal to about 10% by weight of the absorbed fraction of the dose at about 0.4 hours to 5 hours post-injestion, less than or equal to about 50% by weight of the absorbed fraction of the dose at about 3.9 hours to 25 hours post-injection, and less than or equal to about 90% by weight of the absorbed fraction of the dose at about 23 hours to 45 hours post-ingestion.

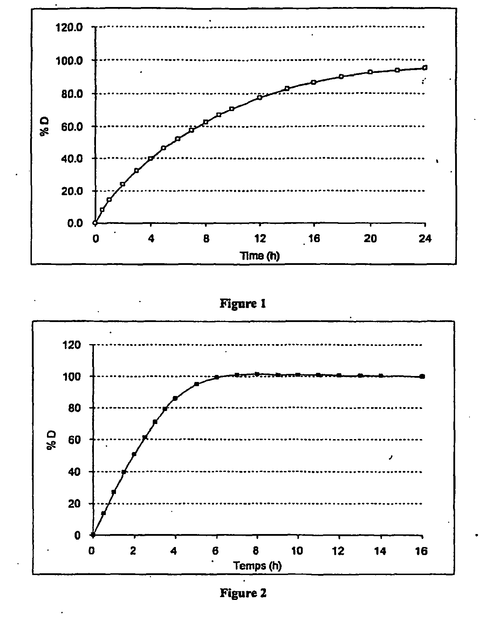

The curve of FIG. 1 of US-B-5,603,957 shows the kinetic profile of the in vivo absorption of acetylsalicylic acid, and more precisely the upper limit of the acetylsalicylic acid in vivo absorption profile induced by the controlled release acetylsalicylic acid microcapsules according to US-B-5,603,957, as a function of time, at a dose of 320 mg. This absorption is expressed in % absorbed relative to the absorbed fraction of the initial dose D. This curve is obtained by conventional deconvolution analysis (Milo GIBALDI and D. PERRIER, Pharmacokinetics, 2nd ed., New York, Marcel Dekker Inc., 1983, p. 145-167) from the mean curves of the plasma concentrations as a function of time after the oral administration of 350 mg of acetylsalicylic acid equivalents of Aspegic® (control form) and 320 mg of acetylsalicylic acid equivalents of microcapsules according to the invention in the form of gelatin capsules. In this case, the tracer molecule chosen for the plasma concentrations as a function of time is necessarily salicylic acid (SA), a metabolite of acetylsalicylic acid. The plasma concentrations of SA are determined by HPLC. The critical points at 0.4, 3.9 and 23 h, given above in the definition of the microcapsules of the invention, are of course to be found on this curve. Beyond this curve, the hepatic acetylsalicylic acid deacetylation mechanism is saturated. It must be considered that all the acetylsalicylic acid in vivo absorption profiles contained in the area under the curve are controlled release acetylsalicylic acid microcapsules according to US-B-5,603,957.

To solve the technical problem on which the invention is based, it is highly preferable in some embodiments that the formulation according to the invention be free or almost free of immediate release acetylsalicylic acid form. The term "almost free" means that the formulation can only include a negligible amount of immediate release acetylsalicylic acid, namely an insufficient amount, so that there is no remaining (non-deacetylated) acetylsalicylic acid in the systemic blood stream after the liver to inhibit COX-I of the systemic blood compartment.

In some embodiments, the oral pharmaceutical composition contains immediate release acetylsalicylic acid. "Immediate release acetylsalicylic acid form" is intended to denote, in the present disclosure, a form in which most of the amount of the acetylsalicylic acid is released, at pH 6.8 and under SINK conditions in an *in vitro* dissolution test, in a relatively brief period of time; for example at least 70% of the acetylsalicylic acid is preferably released in 45 minutes and more preferably in 30 minutes.

All the dissolution profiles to which reference is made in the present disclosure are determined according to the indications of the European Pharmacopoeia, 4th edition, entitled: "Dissolution test for solid dosage forms": type II dissolutest performed under SINK conditions, at 37°C, at a test dose of 10 mg of active, and with agitation of 100 rpm.

The oral pharmaceutical formulation according to the invention contains a dose of between 75 and 310 mg acetylsalicylic acid.

In one embodiment, the oral pharmaceutical composition according to the invention contains the dose of NSAID(s) of between about 1 and 1000 mg, the dose of acetylsalicylic acid in the controlled release acetylsalicylic acid microcapsules is between about 75 and 310 mg, and the composition is a once-a-day administration form. Optionally, the oral pharmaceutical formulation comprises a dose of gastric acid suppressing agent that is between about 5 and 120 mg. The NSAID of the oral pharmaceutical formulation may be in an immediate and/or controlled form. Further, the gastric acid suppressing agent of the oral pharmaceutical formulation may be in an immediate and/or controlled form.

In another embodiment, the oral pharmaceutical composition according the invention contains the dose of NSA1D(s) of between about 1 and 1000 mg, the dose of acetylsalicylic acid in the microcapsules is between about 75 and 310 mg, and the composition is in a twice-a-day administration form. Optionally, the oral pharmaceutical formulation comprises a dose of gastric acid suppressing agent that is between about 5 and 120 mg.

In another embodiment, the oral pharmaceutical formulation according the invention contains the dose of acetylsalicylic acid in the microcapsules of between about 75 and 310 mg, the dose of gastric acid suppressing agent is between about 5 and 120 mg, and the formulation is in a once-a-day administration form.

In another embodiment, the oral pharmaceutical formulation according the invention contains the dose of acetylsalicylic acid in the microcapsules of between about 75 and 310 mg, the dose of gastric acid suppressing agent is between about 5 and 120 mg, and the formulation is in a twice-a-day administration form. Preferably the gastric acid suppressing agent is a proton pump inhibitor. The NSAID of the oral pharmaceutical formulation may be in an immediate and/or controlled form. Further, the gastric acid suppressing agent of the oral pharmaceutical formulation may be in an immediate and/or controlled form.

Some embodiments of the present invention contemplate an oral pharmaceutical formulation designed so that it induces reduction of bleeding and petechia, or ulceration, in the stomach during the treatment.

Further embodiments concern a method for treating a chronic COX-mediated disease or condition, reducing the risk of a thrombotic cardiovascular event in a human patient in need of such treatment and at risk of a thrombotic cardiovascular event, and/or a method of treating a thrombotic disease. The method would comprise the administration to a patient of an oral pharmaceutical formulation according to the instant specification. This method prevents and/or treats pathological disorders associated with excesses of thromboxane, particularly cardiovascular diseases and risks. This method consists in the oral administration of the pharmaceutical formulation according to the invention, preferably in a once or twice-a-day administration.

The inventors find it surprising that they can selectively inhibit COX-1 in the portal vein, permitting minimal aspirin to enter the systemic circulation. This significantly increases the comfort of the patients and the safety of the drug. Patients will be no longer compelled either to interrupt the treatment with aspirin or to switch to another drug. This also permits a method of quickly inhibiting COX in the portal vein, with less side effects than previously known. Finally, the inventors contemplate methods that permit the unexpected precise titration of the effects of aspirin on the liver, the circulatory system, and the stomach.

This remarkable feature gives rise to methods for reducing the side effects during the treatment of diseases at least partially caused by an inhibition of COX in systemic circulation. In some embodiments, this treatment comprises administrating to a patient an oral pharmaceutical composition comprising microcapsules for the controlled release of acetylsalicylic acid in the gastrointestinal environment. The microcapsules of the composition have an *in vitro* release profile, in 0.05M potassium dihydrogenophosphate/sodium hydroxide buffer medium pH 6.8, such that about 70% of the acetylsalicylic acid is released over a period of time of between about 2 and 20 hours, preferably between about 4 and 18 hours, and even more preferably between about 6 and 15 hours. Further, the composition would inhibit COX-1 in portal vein which limits the production of thromboxane while maintaining COX in systemic blood stream, thus limiting the inhibition of the production of prostaglandin and prostacyclin, to protect the gastric endothelium and to maintain its vasodilation properties. Optionally, the formulation further comprises at least one gastric acid suppressing agent increasing the pH of the stomach just enough to minimize the damage resulting from the residual amount of non-deacetylated acetylsalicylic acid entering the systemic blood circulation.

In some embodiments, the oral formulation of the method may comprise at least one active principle for treating a chronic COX-mediated disease or condition to reduce the risk of a thrombotic cardiovascular event in a human patient in need of such treatment and at risk of a thrombotic cardiovascular event. In another embodiment, the oral formulation is used to enhance the safety of antithrombotic treatments.

The inventors have also found that the oral pharmaceutical formulation according to the invention is designed so that it improves the healing process, notably in the stomach during the treatment. The oral pharmaceutical formulation is also designed to decrease other side effects of antithrombotic treatments, such as gastric or intestinal ulceration and hemorrhage, renal failure, edema, atherosclerosis, and any resulting cardiovascular disease.

Other embodiments contemplate methods for improving the healing process of the stomach during the treatment of diseases at least partially caused by an inhibition of COX in systemic circulation. The method of treatment comprises the administration to a patient of an oral pharmaceutical composition including acetylsalicylic acid. Ideally, the oral pharmaceutical composition would be a composition for treating chronic COX-mediated diseases or condition and that would reduce the risk of a thrombotic cardiovascular event in a human patient at risk of a thrombotic cardiovascular event. The composition would therefore comprise at least one active principle for treating a chronic COX-mediated disease or condition and microcapsules for the controlled release of acetylsalicylic acid in the gastrointestinal environment. The microcapsules have an *in vitro* release profile, in 0.05M potassium dihydrogenophosphate/sodium hydroxide buffer medium pH 6.8, such that about 70% of the acetylsalicylic acid is released over a period of time of between about 2 and 20 hours. Further, the composition would inhibit COX in portal vein, which limits the production of thromboxane, while maintaining COX in systemic blood stream and hence limiting the inhibition of the production of prostaglandin and prostacyclin, to protect the gastric endothelium and to maintain its vasodilation properties. Further, the composition may optionally contain at least one gastric acid suppressing agent to increase the pH of the stomach just enough to minimize the damage resulting from the residual amount of non-deacetylated acetylsalicylic acid entering the systemic blood circulation.

In some embodiments, the gastric acid suppressing agent is preferably a proton pump inhibitor. According to the terminology of the present text, the phrase "proton pump inhibitor" used in the singular will designate indifferently one or several proton pump inhibitor, e.g. the lansoprazole, and /or at least one of its metabolites.

In a preferred embodiment of the invention, the oral pharmaceutical formulation according to the invention contains a dose of gastric acid suppressing agent comprised between about 1 and 130 mg. In a more preferred modality of the invention, the oral pharmaceutical formulation according to the invention contains a dose of gastric acid suppressing agent comprised between about 2 and 120 mg.

In some embodiments, controlled release/ immediate release, reference can be made to individually enteric or non enteric coating layered individual units (small beads, granules, microcapsules or pellets). For example, the gastric acid suppressing agent can be designed in the form of controlled release/ immediate release microcapsules, notably of the type of the controlled release/ immediate release acetylsalicylic acid microcapsules, as described herein.

### ACTIVE PRINCIPLES AND NSAID(S)

In some embodiments, the gastric acid suppressing agent is preferably a proton pump inhibitor. A proton pump inhibitor may be a substituted benzimidazole which inhibits gastric acid secretions by specific inhibition of the H⁺, K⁺-ATPase enzymatic system (proton pump) of the secretory surface of parietal gastric cells. A proton pump inhibitor may be an advantageous substitute for histamine H₂ receptor antagonists (blocking of gastric acid secretion) or for antacids, which are not fully effective in the treatment of ulcers, associated or not with *Helicobacter pylori* infection, or of other gastric disorders, and which in addition lead to many side effects.

A proton pump inhibitor may be a lipophilic weak base that is poorly soluble in water. It would therefore undergo rapid degradation under acidic conditions but, would be relatively stable at neutral or basic pH.

In some embodiments, the preferred proton pump inhibitor is a derivative of benzimidazole. This may include, for example, substituted or non substituted benzimidazoles, one or several salts of benzimidazoles, any enantiomer of these benzimidazoles, one or several salts of enantiomer (s), any isomer of these benzimidazoles, any derivative of benzimidazole, any free base of benzimidazole or any mixture of these active principles.

The proton pump inhibitor used in the dosage forms of the invention may be used in neutral form or in the form of an alkaline salt, such as for instance the Mg++, Ca++, Na+, K+or Li+ salts, preferably the Mg++ salts. Where applicable, the compounds listed above may be used in racemic form or in the form of a substantially pure enantiomer thereof, or alkaline salts of the single enantiomers.

In some embodiments, the proton pump inhibitor of the instant invention is one described in pages 7 to 11 of WO-A-97/25066, this extract being incorporated by reference in the present text. In other embodiments, the proton pump inhibitor may be one selected from the WO-A-2004/035020 patent application, which gives also a general formula of the class of benzimidazoles: pages 35-48. This extract of WO-A-2004/035020 is incorporated by reference in the present text.

Examples of proton pump inhibitor may include, but is not limited to, esomeprazole, leminoprazole, omeprazole, pantoprazole, pariprazole, rabeprazole, timoprazole, picoprazole and tenatoprazole.

Other suitable proton pump inhibitors are disclosed in EP-A1-0005129, EP-A1-174 726, EP-A1-166 287, GB 2 163 747 and WO90/06925, WO91/19711, WO91/19712, and further especially suitable compounds are described in WO95/01977 and WO094/27988.

The gastric acid suppressing agent is preferably a proton pump inhibitor, but H₂ receptor antagonists such as ranitidine, cimetidine or famotidine may be used in the pharmaceutical compositions with an alginate as proposed in WO 95/017080 or together with antacid agent(s). A wide variety of antacid agent(s) and/or alginates may be used in combination with a suitable proton pump inhibitor in the fixed unit dosage form according to the present invention. Such antacid agents include for example aluminum hydroxide, calcium carbonate, magnesium hydroxide, magnesium carbonate and aluminum magnesium hydroxide carbonate (hydrotalcit) taken alone or in combinations with each other. The alginates may be an alginate selected from alginic acid or sodium alginate or other pharmaceutically acceptable alginate salts, hydrates, esters etc. Especially preferred antacid agents are magnesium or calcium based antacid agents and aluminum hydroxide/magnesium carbonate complex. Suitable antacid agents are for instance described in U.S. Pat. No. 5,409,709.

In yet other embodiments, the preferred proton pump inhibitor is in the form of a racemate, an alkaline salt or one of its single enantiomers, optionally in combination with antacid agent(s), and can be an immediate release form and/or a controlled release form.

In other embodiments, the oral pharmaceutical formulation according to the invention comprises at least another third active principle different from acetylsalicylic acid and from gastric acid suppressing agent. In some embodiments, third active principle is selected in the group of the anti-inflammatory drugs. In some embodiments, the second active principle is a NSAID.

In some embodiments, the second active principle is a cardiovascular drug. The cardiovascular drug may be selected from, but not limited to, anti-platelet drugs, beta adrenergic receptor blockers, calcium channel blockers, angiotensin converting enzyme inhibitors, diuretics, anti-arrhythmic drugs, anti-ischemic drugs, anti-hypertensive drugs, beta adrenergic agonists, cardiac glycosides, nitrates, sodium channel blockers, central nervous system acting anti-hypertensive drugs, potassium channel activators, vasodilators, vasoconstrictive drugs, and mixtures thereof.

In other embodiments, the oral pharmaceutical formulation according to the invention comprises at least another third active principle different from acetylsalicylic acid and from the first active principle. In some embodiments, this third active principle is an anti-inflammatory drug. In some embodiments, this third active principle is selected in the group comprising, anti-platelet drugs, beta adrenergic receptor blockers, calcium channel blockers, angiotensin converting enzyme inhibitors, diuretics, anti-arrhythmic drugs, anti-ischemic drugs, anti-hypertensive drugs, beta adrenergic agonists, cardiac glycosides, nitrates, sodium channel blockers, central nervous system acting anti-hypertensive drugs, potassium channel activators, vasodilators, vasoconstrictive drugs, and mixtures thereof.

Examples of anti-platelet drugs include non-steroidal anti-inflammatory drugs, dipyridamole, and ticlopidine.

Examples of diuretics include acetazolamide, dichlorphenamide, methazolamide, glycerin, isosorbide, mannitol, urea, furosemide, bumetanide, ethacrynic acid, torsemide, azosemide, muzolimine, piretanide, tripamide, bendroflumethiazide, benzthiazide, chlorothiazide, hydrochlorothiazide, hydroflumethiazide, methyclothiazide, polythiazide, trichlormethiazide, chlorthalidone, indapamide, metolazone, quinethazone, amiloride, triamterene, spironolactone, canrenone, potassium canrenoate.

Examples of angiotensin converting enzyme inhibitors include benazepril, captopril, enalapril, fosinopril sodium, lisinopril, quinapril, ramipril, spirapril.

Examples of nitrates include amyl nitrite (isoamyl nitrite), nitroglycerin, isosorbide dinitrate, isosorbide-5-mononitrate, erythrityl tetranitrate.

Examples of calcium channel blockers include amlodipine, bepridil, diltiazem, felodipine, isradipine, nicardipine, nifedipine, nimodipine, verapamil. Examples of vasodilator drugs include nitrovasodilators such as nitroglycerin, isosorbide dinitrate, sodium nitroprusside; angiotensin receptor antagonists such as losartan, phosphodiesterase inhibitors such as amrinone, milrinone, and vesnarinone; "direct" vasodilators such as hydralazine, nicorandil, adrenergic receptor antagonists such as prazosin, and other quinazoline derivatives, phentolamine, labetalol, carvedilol, and bucindolol; Ca²⁺ channel blocking drugs such as nifedipine, amlodipine, and sympathomimetics such as dobutamine.

Examples of anti-arrhythmic drugs include adenosine, amiodarone, bretylium, digoxin, digitoxin, diltiazem, disopyramide, esmolol, flecainide, lidocaine, mexiletine, moricizine, phenytoin, procainamide (N-acetyl procainamide), propafenone, propranolol, quinidine, sotalol, tocainide, verapamil.

According to another embodiment, the second active principle is an anti-diabetic drug. Examples of anti-diabetic drugs include: acarbose, acetohexamide, buformin, 1-butyl-3-metanilylurea, carbutamide, chlorpropamide, ciglitazone, glibornuride, gliclazide, glimepiride, glipizide, gliquidone, glisoxepid, glyburide, glybuthiazole, glybuzole, glyhexamide, glymidine, glypinamide, metformin, miglitol, nateglinide, phenbutamide, phenformin, pioglitazone, proinsulin, repaglinide, rosiglitazone, tolazamide, tolbutamide, tolcyclamide, troglitazone and/or the pharmaceutical salts and/or the complexes and/or the prodrugs and/or mixtures thereof.

There is a great need to combine anti-platelet aggregation drugs to NSAIDs, particularly COX-2 inhibitor anti-inflammatory drugs, without inducing gastric side effects. A very large number of patients who are treated with common NSAIDs, or with more specific NSAIDs such as COX-2 inhibitors, have severe side effects, including life-threatening ulcers and thrombotic cardiovascular events, that limit the therapeutic potential of said drugs. In addition, there is evidence that patients with chronic inflammatory conditions, such as rheumatoid arthritis and systemic lupus erythematosis are at increased risk for thrombotic cardiovascular events. Thus, it is desirable that these patients receive antiplatelet therapy, with only minimal side effects. This need is reinforced by the fact that many patients treating with NSAIDs or suffering from chronic COX-mediated disease or condition, are elderly and thus are at increased risk for thrombotic cardiovascular events. It may be possible to associate low dose aspirin with COX-2 inhibitors; however, due to the aspirin dilemma, the gastro-protection activity of the prostacyclin and prostaglandins is affected and thus it induces severe gastric disorders. Some embodiments of the instant invention offer an advantageous solution to this problem.

Examples of NSAID(s) include, but are not limited to, aminoarylcarboxylic acid and its derivatives such as: enfenamic acid, flufenamic acid, isonixin, meclofenamic acid, mefenamic acid, morniflumate, niflumic acid & tolfenamic acid. Other examples of NSAID(s) include, but are not limited to, arylacetic acid and its derivatives, aceclofenac, acemetacin, amfenac, bromfenac, cimmetacin, diclofenac, etodolac, fentiazac, glucametacin, indomethacin, lonazolac, metiavinic acid, oxametacine, pirazolac, proglumetacin, sulindac, tiaramide, tolmetin and zomepirac. Other examples of NSAID(s) include, but are not limited to arylcarboxylic acids such as ketorolac and tinoridine, arylpropionic acid and its derivatives, alminoprofen, bermoprofen, carprofen, dexibuprofen, fenbufen, fenoprofen, flunoxaprofen, flurbiprofen, ibuprofen, ibuproxam, ketoprofen, loxoprofen, naproxen, oxaprozin, pranoprofen, protizinic acid & tiaprofenic acid, pyrazoles, pyrazolones, benzpiperylon, mofebutazone, oxyphenbutazone, phenylbutazone & ramifenazone, salicylic acid derivatives, acetaminosalol, benorylate, eterisalate, fendosal, imidazole salicylate, lysine acetylsalicylate, morpholine salicylate, parsalmide, salamidacetic acid & salsalate, thiazinecarboxamides, ampiroxicam, droxicam, lornoxicam, meloxicam, piroxicam & tenoxicam, bucillamine, bucolome, bumadizon, diferenpiramide, ditazol, emorfazone, nabumetone, nimesulide, proquazone and piroxicam. Other examples of NSAID(s) include, but are not limited to, alclofenac, azapropazone, benoxaprofen, bucloxic acid, choline magnesium trisalicylate, clidanaque, clopinaque, dapsone, diflunisal, fenclofenec, floctafenine, flufenisal, (r)-flurbiprofen, (s)-flurbiprofen, furofenaque, feprazone, fluprofen, ibufenaque, indoprofen, isoxepac, isoxicam, miroprofen, mefenamic, meclofen, niflumic acid, nitroflurbiprofen, oxipinaque, podophyllotoxin derivatives, piprofen, pirprofen, prapoprofen, sudoxicam, suprofen, tiaprofenic acid, tiopinac, tioxaprofen, zidometacin, 2-fluoro-a-methyl[1,1'-biphenyl]-4-acetic acid 4-(nitrooxy)butyl ester, ketoprofen, ketorolac and/or mixtures thereof.

More preferably, the NSAID(s) is selected from the following: lornoxicam, diclofenac, nimesulide, ibuprofen, piroxicam, piroxicam (betacyclodextrin), naproxen, ketoprofen, tenoxicam, aceclofenac, indometacin, nabumetone, acemetacin, morniflumate, meloxicam, flurbiprofen, tiaprofenic acid, proglumetacin, mefenamic acid, fenbufen, etodolac, tolfenamic acid, sulindac, phenylbutazone, fenoprofen, tolmetin, dexibuprofen and/or the pharmaceutical salts and/or the complexes and/or the prodrugs and/or mixtures thereof.

The COX-2 specific or selective inhibitors rofecoxib, etoricoxib, celecoxib, valdecoxib, parecoxib, COX-189 (Novartis), BMS347070 (Bristol Myers Squibb), tiracoxib , ABT963 (Abbott), CS502 (Sankyo), GW406381 (GlaxoSmithKline), and/or mixtures thereof.

### MICROCAPSULE COATING

In the disclosure of the invention, the term "controlled release acetylsalicylic acid microcapsules" denotes microparticles of acetylsalicylic acid that are film-coated with at least one coating for modified/controlled release of ASA. The non-film-coated microparticles of acetylsalicylic acid may, for example, be neutral cores coated with at least one layer containing ASA, or microparticles of pure acetylsalicylic acid or alternatively granules formed by a matrix of support excipients including lansoprazole.

The controlled release acetylsalicylic acid microcapsules act as vehicles for the transport and the release of acetylsalicylic acid and, optionally, of one or more other active principles in the stomach and in the small intestine. Advantageously, the coating has sufficient mechanical strength to prevent it tearing and/or breaking up in the organism, until the end of the release of the active principle.

The coating of the controlled-release acetylsalicylic acid microcapsules comprises at least one layer which controls the modified release, where the composition of said layer contains at least one film-forming (co)polymer that is insoluble in the fluids of the gastrointestinal tract, at least one (co)polymer that is soluble in the fluids of the gastrointestinal tract and at least one plasticizer.

The one film-forming (co)polymer that is insoluble in the fluids of the gastrointestinal tract may include, but is not limited to, non-water-soluble derivatives of cellulose, ethylcellulose, cellulose acetate, polyvinyl acetates, and mixtures thereof.

The (co)polymer that is soluble in the fluids of the gastrointestinal tract may include, but is not limited to, nitrogenous (co)polymers, polyacrylamides, poly-N-vinylamides, polyvinylpyrrolidones (PVP), poly-N-vinyllactams, water-soluble derivatives of cellulose, polyvinyl alcohols (PVAs), polyoxyethylenes (POEs), and mixtures thereof. Preferably, the polymer is polyvinylpyrrolidone.

The plasticizer may include, but is not limited to, cetyl alcohol esters, glycerol and its esters, acetylated glycerides, glyceryl monostearate, glyceryl triacetate, glyceryl tributyrate, phthalates, dibutyl phthalate, diethyl phthalate, dimethyl phthalate, dioctyl phthalate, citrates, acetyl tributyl citrate, acetyltriethyl citrate, tributyl citrate, triethyl citrate, sebacates, diethyl sebacate, dibutyl sebacate, adipates, azelates, benzoates, plant oils, fumarates, diethyl fumarate, malates, diethyl malate, oxalates, diethyl oxalate, succinates, dibutyl succinate, butyrates, triacetin, malonates, diethyl malonate, castor oil and mixtures thereof. Preferably, the plasticizer is castor oil.

Optionally, the layer also contains at least one water-insoluble hydrophilic film-forming (co)polymer that is insoluble in the fluids of the gastrointestinal tract, carrying groups that are ionized in the fluids of the gastrointestinal tract. The water-insoluble hydrophilic film-forming (co)polymer that is insoluble in the fluids of the gastrointestinal tract may include, but is not limited to, water-insoluble charged acrylic derivatives, (co)polymers of acrylic and methacrylic acid ester carrying at least one quaternary ammonium group. Further, the polymer may contain at least one copolymer of alkyl (meth)acrylate and of trimethylammonioethyl methacrylate chloride, and more precisely the products sold under the trade marks Eudragit ® RS and/or Eudragit ® RL, e.g. the powders Eudragit ® RL PO and/or Eudragit ® RS PO and/or the granules Eudragit ® RL 100 and/or Eudragit ® RS 100 and/or the suspensions and/or solutions of these Eudragit ® RL and Eudragit ® RS, namely, respectively, Eudragit® RL 30D and/or Eudragit® RS 30D and/or Eudragit® RL 12.5, Eudragit® RS 12.5, and mixtures thereof.

Optionally, the layer also contains at least one surfactant and/or lubricant. The surfactant and/or lubricant may include, but is not limited to, anionic surfactants, alkali metal or alkaline-earth metal salts of fatty acids, stearic acid, oleic acid, nonionic surfactants, polyoxyethylenated oils, polyoxyethylenated hydrogenated castor oil, polyoxyethylene-polyoxypropylene copolymers, polyoxyethylenated esters of sorbitan, polyoxyethylenated derivatives of castor oil, stearates, calcium stearate, magnesium stearate, aluminum stearate or zinc stearate, stearyl fumarates, preferably sodium stearyl fumarate, glyceryl behenates, and mixtures thereof.

In one preferred embodiment, the composition of the modified-release layer contains at least one film-forming polymer(s) present in a proportion of about 10 to 90%, preferably about 20 to 40% by weight on a dry basis relative to the total mass of the coating composition; at least one water-insoluble hydrophilic film-forming polymer(s) present in a proportion of 10 to 90%, preferably about 20 to 40% by weight on a dry basis relative to the total mass of the coating composition; at least one polymer(s) that is soluble in the fluids of the gastrointestinal tract is present in a proportion of about 2 to 25%, preferably about 5 to 15% by weight on a dry basis relative to the total mass of the coating composition; and at least one plasticizer present in a proportion of about 2 to 20%, preferably of about 4 to 15% by weight on a dry basis relative to the total mass of the coating composition. Optionally, the composition also contains at least one surfactant and/or lubricant present in a proportion of about 2 to 20%, preferably of about 4 to 15% by weight on a dry basis relative to the total mass of the coating composition.

In some embodiments, particular qualitative and quantitative details regarding at least some of the constituents of this coating composition, are found in, for example, European patent EP-B-0 709 87 or PCT applications WO-A-2004/010983 and WO-A-2004/010984.

The controlled-release acetylsalicylic acid microcapsules have an *in vitro* release profile such that in 0.05M potassium dihydrogenophosphate/sodium hydroxide buffer medium pH 6.8, up to 70% of the acetylsalicylic acid is released over a period of time of between 2 and 8 hours, and more preferably between about 2 and about 6 hours, and up to 40% of the acetylsalicylic acid is released over a period of time of between about 0.5 and about 5 hours, preferably between about 1 and about 4 hours, and even more preferably between about 1 and about 3 hours.

In some embodiments, the controlled-release acetylsalicylic acid microcapsules have an *in vitro* release profile such that in a 0.04M hydrochloric acid medium pH 1.4, up to 40% of the acetylsalicylic acid is released over a period of time of less than or equal to about 3 hours, preferably less than or equal to about 2 hours, and even more preferably less than or equal to about 0.75 hours.

According to another pharmacokinetic definition of the pharmaceutical formulation, the controlled-release acetylsalicylic acid microcapsules have an in vitro release profile in 0.05M potassium dihydrogenophosphate/sodium hydroxide buffer medium pH 6.8, such that, for any value of time t of between 2h and t(70%), preferably for any value of time t of between 1h and t(70%), the % of dissolved (released) acetylsalicylic acid is greater than or equal to 35xt / t(70%).

According to the invention, the proportion of acetylsalicylic acid in the microcapsules (expressed as % acetylsalicylic acid by weight on a dry basis relative to the total mass of the microcapsules) is between about 5 and 80, preferably between about 10 and 60, and even more preferably between about 20 and 50.

In some preferred embodiments, the coating of the controlled release acetylsalicylic acid microcapsules represents 5 to 50% by weight, of the total mass of said microcapsules. In other preferred embodiments, the microcapusules represents at most 40% by weight on a dry basis, of the total mass of the microcapsules. In other preferred embodiments, the coating of the microcapsules represents at most 15%, of the total weight of the microcapsules, by weight on a dry basis of the microcapsules.

In some embodiments, the coating of each controlled-release acetylsalicylic acid microcapsule in the pharmaceutical formulation has a coating that is nonenteric and does not disintegrate based upon the pH.

In some embodiments, the diameter of the controlled release acetylsalicylic acid microcapsules is less than or equal to about 1000 µm, preferably between about 50 and 800 µm, and even more preferably between about 100 and 600 µm. This size is advantangeous because it makes it possible for the microcapsules to cross the stomach independently of the opening of the pylorus. The gastric transit time is thus more uniform. The microparticle diameters to which the present disclosure refers are, unless otherwise indicated, mean diameters by volume.

In some embodiments, the controlled release acetylsalicylic acid microcapsules are obtained from particles of acetylsalicylic acid having a size of between 250 and 800 µm before the coating operation.

The controlled release acetylsalicylic acid microcapsules may be obtained from particles of acetylsalicylic acid which are coated by being sprayed with the intimate combination forming the coating, suspended in an organic solvent or mixture of organic solvents. The coating process, which constitutes a further subject of the invention, fits into the general pattern of microencapsulation techniques, of which the main ones are summarized in the article by C. DUVERNEY and J. P. BENOIT in "L'actualité chimique", December 1966. More precisely, the technique in question is microencapsulation by film coating. Preferably, this process consists essentially in: preparing the coating composition in a solvent system, applying the composition/solvent system mixture to particles of acetylsalicylic acid, drying the resulting microcapsules, and if appropriate, mixing the latter with at least one anticaking agent. Examples of solvents which are suitable for forming part of the composition of the solvent system are ketones, esters, chlorinated solvents, alcohols, preferably aliphatic alcohols, alkanes or mixtures thereof. These solvents are advantageously C₁-C₆ compounds and particularly preferably acetone, methyl ethyl ketone, methanol, ethanol, isopropanol, cyclohexane and methylene chloride. If the coating methodology which can be used according to the invention is considered in greater detail, it can be stated that the coating composition/solvent system mixture is applied by being sprayed onto the moving particles of ASA, said movement preferably being created by mechanical agitation or by blowing (fluidization). To obtain microcapsules according to some embodiments of the invention possessing the desired absorption kinetics, it is necessary to encapsulate particles of acetylsalicylic acid with a mean size of between 75 and 500 µm, preferably of between 300 and 500 µm, for a dose of between 75 and 320 mg.

According to a particular embodiment of the invention, the controlled-release acetylsalicylic acid microcapsule coating consists of a single coating layer or a single coating film. This simplifies their preparation and limits the degree of coating.

The monolayer or multilayer coating may comprise various other additional adjuvants conventionally used in the coating field. They may be, for example, pigments or coloring agents, fillers, or anti-foaming agents. To prevent the problems of caking of the coated microparticles, the inventors contemplate adding to the microcapsules at least one anticaking agent preferably formed of talc, colloidal silica or a mixture of the two.

The controlled-release acetylsalicylic acid microcapsules can be used for the preparation of novel pharmaceutical forms of aspirin having a biochemical selectivity for the inhibition of thromboxane relative to the other prostaglandins. In particular, the microcapsules may be used for the preparation of novel pharmaceutical forms useful as platelet aggregation inhibitors. Furthermore, the microcapsules can be used for the preparation of novel pharmaceutical forms active in the prevention and/or treatment of cardiovascular diseases and risks.

Advantageously, these formulation units are novel in their structure, their presentation and their composition. The formulation units may be presented in the form of a sachet of powder, a sachet of a powder for multidose suspension to be reconstituted, a tablet or a gelatin capsule. They can contain, for instance, a dose of acetylsalicylic acid of 50 to 325 mg of acetylsalicylic acid and a dose of proton pump inhibitor of about 1 to 300 mg, preferably about 2 to 200 mg and particularly preferably about 5 to 120 mg. Such pharmaceutical forms are preferably administered in single or twice daily doses.

In some embodiments, one may mix, in one and the same gelatin capsule, tablet or powder, at least two types of microcapsules whose absorption kinetics are different but within the framework characteristic of the controlled-release acetylsalicylic acid microcapsules according to US-B-5,603,957 (profile of curve of Fig. 1).

The invention will be understood more clearly from the following Examples, which are given solely by way of illustration and serve to provide a clear understanding of the invention and to illustrate its different embodiments and/or modes of implementation, as well as its various advantages.

### EXAMPLES

### Reference Example 1 Preparation of controlled-release aspirin-based microcapsules

66 g of ethyl cellulose (Ethocel 7 Premium / Dow), 7 g of Plasdone K29/32® (povidone/ISP), 8 g of castor oil, 9 g of magnesium stearate and 10 g tartaric acid are dispersed in 1200 g of a mixture made of 60% of isopropanol & 40% of acetone. The suspension is sprayed on 900 g of acetylsalicylic acid (aspirin) crystals, previously sieved between 200 and 500 µm.

These microcapsules have been tested in a pH 6.8 (KH₂PO₄ 0.05M/ NaOH) dissolution medium maintained at 37°C and stirred with a paddle speed of 100 rpm (USP II apparatus) (See Figure 1).

### Reference Example 2 Preparation of controlled release omeprazole based microcapsules

Step 1 : 700 g of omeprazole and 100 g de Klucel EF® (hydroxypropyl cellulose /Aqualon) are dispersed in 3000 g of isopropanol. The suspension is sprayed on 200 g of neutral microspheres (Asahi-Kasei) in a spray coater Glatt GPCG1.

Step 2 : 50 g of ethyl cellulose (Ethocel 20 Premium / Dow), 20 g of Plasdone K29/32® (povidone/ISP), 20 g of Lutrol F-68 (poloxamer 188 / BASF) and 10 g of castor oil are dispersed in mixture made of 60% of isopropanol and 40% of acetone. This solution is sprayed on 900 g of omeprazole granules (prepared at step 1).

The obtained microparticles are filled into a size 3 gelatin capsule . The dose of omeprazole per capsule is, in this test, 80 mg i.e. 127 mg of microcapsules. These microcapsules have been tested in a pH 6.8 (KH₂PO₄ 0.05M/ NaOH) dissolution medium maintained at 37°C and stirred with a paddle speed of 100 rpm (USP II apparatus). (See Figure 2).

### Reference Example 3: Preparation of immediate release lansoprazole- based microcapsules

900 g of lansoprazole & 100 g of Klucel EF® (hydroxypropyl cellulose / Aqualon) are previously dry-mixed in a high shear granulator (Aeromatic PMA1) for 5 minutes. This mixture is then granulated with water (180 g). The granules are dried at 40°C in ventilated oven, and calibrated on 500 µm sieve. The fraction 200-500 µm is selected by sieving.

These microcapsules have been tested in a pH 6.8 (KH₂PO₄ 0.05M/ NaOH) dissolution medium maintained at 37°C and stirred with a paddle speed of 100 rpm (USP II apparatus) Their release is immediate.

### Reference Example 4: Capsule containing controlled release aspirin and controlled release omeprazole

180 mg of microcapsules of acetylsalicylic acid prepared in example 1 (i.e. 162.5 mg of acetylsalicylic acid) and 15.4 mg of microcapsules of omeprazole (i.e. 10 mg of omeprazole) prepared at example 2 are filled in size 2 capsule.

This capsule is the final form of the drug for preventing cardiovascular diseases (by means of aspirin), while hindering gastric damages due to the presence of a proton pump inhibitor and controlled release-ASA microcapsules.

### Reference Example 5: Capsule containing controlled release aspirin and immediate release lansoprazole

180 mg of microcapsules of acetylsalicylic acid prepared in example 1 (i.e. 162.5 mg of acetylsalicylic acid) and 5.5 mg of microcapsules of lansoprazole (i.e. 5 mg of lansoprazole) prepared as in example 3 are filled in size 2 capsule.

This capsule is the final form of the drug for preventing cardiovascular diseases (by means of aspirin), while hindering gastric damages due to the presence of a proton pump inhibitor and controlled release acetylsalicylic acid microcapsules.

### Reference Example 6: Capsule containing controlled release aspirin and enteric coated lansoprazole

180 mg of microcapsules of acetylsalicylic acid prepared in example 1 (i.e. 162.5 mg of acetylsalicylic acid) and 6.2 mg of microcapsules of lansoprazole (i.e. 5 mg of lansoprazole) prepared at example 4 are filled in size 2 capsule.

This capsule is the final form of the drug for preventing cardiovascular diseases (by means of aspirin), while hindering gastric damages due to the presence of a proton pump inhibitor and controlled release acetylsalicylic acid microcapsules.

### Reference Example 7: Preparation of immediate release celecoxib based microcapsules

860 g of celecoxib, 70 g of Klucel EF® (hydroxypropyl cellulose / Aqualon) & 70 g of Lutrol F-68 (poloxamer 188 / BASF) are previously dry-mixed in a high shear granulator (Aeromatic PMA1) for 5 minutes. This mixture is then granulated with water (180 g). The granules are dried at 40°C in ventilated oven, and calibrated on 500 µm sieve. The fraction 200-500 µm is selected by sieving.

These microcapsules have been tested in a pH 6.8 (KH₂PO₄ 0.05M/ NaOH) dissolution medium maintained at 37°C and stirred with a paddle speed of 100 rpm (USP II apparatus) Their release is immediate.

### Reference Example 8: Preparation of immediate release rofecoxib based microcapsules

1500 g of rofecoxib, 150 g de Klucel EF® (hydroxypropyl cellulose / Aqualon) and 150 g of Cremophor RH 40® (PEG 40-hydrogenated castor oil / BASF) are dispersed in 4000 g of water. The suspension is sprayed on 200 g of neutral microspheres (Asahi-Kasei) in a spray coater Glatt GPCG1.

These microcapsules have been tested in a pH 6.8 (KH₂PO₄ 0.05M/ NaOH) dissolution medium maintained at 37°C and stirred with a paddle speed of 100 rpm (USP II apparatus). Their release is immediate.

### Reference Example 9: Preparation of immediate release meloxicam based microcapsules

600 g of meloxicam, 100 g de Klucel EF® (hydroxypropyl cellulose / Aqualon) and 100 g of Lutrol F-68 (poloxamer 188 / BASF) are dispersed in 2000 g of water. The suspension is sprayed on 200 g of neutral microspheres (Asahi-Kasei) in a spray coater Glatt GPCG1.

These microcapsules have been tested in a pH 6.8 (KH₂PO₄ 0.05M/ NaOH) dissolution medium maintained at 37°C and stirred with a paddle speed of 100 rpm (USP II apparatus). Their release is immediate.

### Reference Example 10: Preparation of enteric coated lansoprazole based microcapsules

Step 1: 900 g of lansoprazole & 100 g of Klucel EF® (hydroxypropyl cellulose /Aqualon) are previously dry-mixed in a high shear granulator (Aeromatic PMA1) for 5 minutes. This mixture is then granulated with water (180 g). The granules are dried at 40°C in ventilated oven, and calibrated on 500 µm sieve. The fraction 200-500 µm is selected by sieving.

Step 2: 50 g of Eudragit® L100-55® (Rohm) and 10 g of triethyl citrate are dispersed in isopropanol. This solution is sprayed on 450 g of lansoprazole granules (prepared at step 1).

These microcapsules have been tested in a pH 6.8 (KH₂PO₄ 0,05M/ NaOH) dissolution medium maintained at 37°C and stirred with a paddle speed of 100 rpm (USP II apparatus) Their release is immediate.

### Example 11: Capsule containing controlled release aspirin and immediate release celecoxib

180 mg of microcapsules of acetylsalicylic acid prepared in example 1 (i.e. 162.5 mg of acetylsalicylic acid) and 233 mg of microcapsules of celecoxib (i.e. 200 mg of celecoxib) prepared at example 7 are filled in size 0 capsule.

This capsule is the final dosage form for preventing cardiovascular diseases (by means of aspirin), said diseases being caused by repeated administration of anti-inflammatory drugs, such as COX-2 inhibitors (celecoxib).

### Example 12: Capsule containing controlled release aspirin and immediate release rofecoxib

180 mg of microcapsules of acetylsalicylic acid prepared in example 1 (i.e. 162.5 mg of acetylsalicylic acid) and 33 mg of microcapsules of rofecoxib (i.e. 25 mg of rofecoxib) prepared at example 8 are filled in size 2 capsule.

This capsule is the final dosage form for preventing cardiovascular diseases (by means of aspirin), said diseases being caused by repeated administration of anti-inflammatory drugs, such as COX-2 inhibitors (rofecoxib).

### Reference Example 13: Capsule containing controlled release aspirin and immediate release meloxicam

180 mg of microcapsules of acetylsalicylic acid prepared in example 1 (i.e. 162.5 mg of acetylsalicylic acid) and 25 mg of microcapsules of meloxicam (i.e. 15 mg of meloxicam) prepared at example 9 are filled in size 2 capsule.

This capsule is the final dosage form for preventing cardiovascular diseases (by means of aspirin), said diseases being caused by repeated administration of anti-inflammatory drugs, such as COX-2 inhibitors (meloxicam).

### Example 14: Capsule containing controlled release aspirin, enteric coated lansoprazole and immediate release celecoxib

180 mg of microcapsules of acetylsalicylic acid prepared in example 1 (i.e. 162.5 mg of acetylsalicylic acid), 6.2 mg of microcapsules of lansoprazole (i.e. 5 mg of lansoprazole) prepared at example 10 and 233 mg of microcapsules of celecoxib (i.e. 200 mg of celecoxib) prepared at example 7 are filled in size 0 capsule.

This capsule is the final dosage form for preventing cardiovascular diseases (by means of aspirin), said diseases being caused by repeated administration of anti-inflammatory drugs, such as COX-2 inhibitors (celecoxib), while hindering gastric damages due to the presence of a proton pump inhibitor and controlled release acetylsalicylic acid microcapsules. The combination of a proton pump inhibitor with the controlled release acetylsalicylic acid microcapsules makes it possible to prevent gastric damages due to aspirin.

### Example 15 showing that the known controlled release acetylsalicylic acid microcapsules could be improved, by means of the combination according to the invention:

Controlled release microcapsules of aspirin according to example 1 were compared to aspirin at a dose of 325 mg in double blind, randomized cross-over study, on 24 healthy non smoking volunteers. Endoscopic damage was assessed on days 0, 7, 14, 21 on each treatment period. The primary end point was the total number of gastroduodenal erosions and petechiae assessed endoscopically. The study performed by a group led by Professor Hawkey of Gastroenterology Division of University Hospital, Nottingham (UK) indicated that Controlled released aspirin cause less endoscopic damage than conventional aspirin.

The study indicated that significantly fewer gastric lesions were observed in patients taking controlled released aspirin 325 mg than in patients taking entero-coated aspirin at the same dose. In particular, gastric erosion per patient were 1.57 with Controlled released aspirin 325 compared to 5.48 with entero-coated aspirin product, or a reduction of 70% (p<0.001). Concerning haemorrhagic event, 0.3 events per patient were observed with controlled released aspirin 325 compared to 2.96 with conventional aspirin (p<0.001). In addition, 3.09 petechia per patient were observed with controlled released aspirin compared to 7.35 with the comparator (p<0.001).

These very positive results for controlled released aspirin could be explained by the biochemical selectivity of the product which inhibits platelets COX-1 and consequently thromboxan (TXB2), the platelet aggregant prostanoids, while sparing prostacyclin (PGI2), the systemic cytoproctective prostaglandin generated by endothelial COX-1.

Although, this result is very positive for GI tract safety, it still leaves some room for improvement of the controlled released aspirin directed toward a decrease in the frequency of gastro intestinal tract side effects. It is worthwhile noticing that if the number of adverse events is significantly decreased using Controlled released aspirin compared to conventional aspirin, the GI tract events (i.e. erosion and petechia) are still measurable and could lead to some safety concerns for chronic use.

## Claims

1. An oral pharmaceutical formulation comprising:
• at least one cyclooxygenase-2 selective inhibitor selected from the group consisting of rofecoxib, etoricoxib, celecoxib, valdecoxib, parecoxib, COX-189, BMS347070, tiracoxib, ABT963, CS502 and GW406381, and
• acetylsalicylic acid that is coated with a coating composition to form microcapsules,
wherein said coating, composition comprises:
- at least one film-forming (co)polymer that is insoluble in the fluids of the gastrointestinal tract
- at least one (co)polymer that is soluble in the fluids of the gastrointestinal tract; and
- at least one plasticizer,
and said coating is nonenteric and does not disintegrate based upon the pH.
wherein the amount of acetylsalicylic acid in said formulation is between 75 and 310mg, and said microcapsules have a release profile such that 70% of the acetylsalicylic acid in 0.05M potassium dihydrogenophosphate/sodium hydroxide buffer medium at a pH of 6.8 is released between 2 and 20 hours, determined according to the indications of the European Pharmacopoeia, 4^{th} edition, entitled "Dissolution test for solid dosage forms": type II dissolutest performed under SINK conditions, at 37°C, at a test dose of 10 mg of active, and with agitation of 100 rpm, whereby when ingested in a single administration the formulation induces controlled acetylsalicylic acid absorption kinetics in vivo, extending over at least 24 hours, the acetylsalicylic acid absorption being: less than or equal to 10% by weight of the absorbed fraction of the dose at 0.4 hours post ingestion, less than or equal to 50% by weight of the absorbed fraction of the dose at 3.9 hours post ingestion, and less than or equal to 90% by weight of the absorbed fraction of the dose at 23 hours post ingestion.

2. The oral pharmaceutical formulation according to claim 1, wherein:
(i) said film-forming (co)polymer is selected from the group consisting of: non-water-soluble derivatives of cellulose, polyvinyl acetates, and mixtures thereof;
(ii) said soluble (co)polymer is selected from the group consisting of: nitrogenous copolymers, water-soluble derivatives of cellulose, polyvinyl alcohols, polyoxyethylenes, and mixtures thereof; and
(iii) said plasticizer is selected from the group consisting of: cetyl alcohol esters, glycerol, glycerol esters, phthalates, citrates, sebacates, adipates, azelates, benzoates, plant oils, fumarates, malates, oxalates, succinates, butyrates, triacetin, malonates, castor oil, and mixtures thereof.

3. The oral pharmaceutical formulation according to claim 1, wherein:
(i) said film-forming polymer is present in a proportion of 10 to 90%, by weight on a dry basis relative to the total mass of said coating composition;
(ii) said soluble (co)polymer is present in a proportion of 2 to 25%, by weight on a dry basis relative to the total mass of said coating composition; and
(iii) said plasticizer is present in a proportion of 2 to 20%, by weight on a dry basis relative to the total mass of said coating composition.

4. The oral pharmaceutical formulation according to claim 1, wherein said coating composition further comprises:
(iv) a second film-forming (co)polymer that is hydrophilic and water-insoluble and carries groups that are ionized in the fluids of the gastrointestinal tract.

5. The oral pharmaceutical formulation according to claim 4, wherein said second film-forming (co)polymer is a water-insoluble, charged acrylic derivative.

6. The oral pharmaceutical formulation according to claim 4, wherein said second film-forming (co)polymer is present in a proportion of 0 to 90%, by weight on a dry basis relative to the total mass of said coating composition.

7. The oral pharmaceutical formulation according to claim 1, wherein said coating composition further comprises:
(v) at least one surfactant and/or lubricant.

8. The oral pharmaceutical formulation according to claim 7, wherein the at least one surfactant is selected from the group consisting of: anionic surfactants, nonionic surfactants, and mixtures thereof.

9. The oral pharmaceutical formulation according to claim 7, wherein the surfactant and/or lubricant is present in a proportion of 2 to 20%, by weight on a dry basis relative to the total mass of said coating composition.

10. The oral pharmaceutical formulation according to claim 1, further comprising a proton pump inhibitor.

11. The oral pharmaceutical formulation according to claim 10, wherein the proton pump inhibitor is selected from the group consisting of esomeprazole, leminoprazole, omeprazole, pantoprazole, pariprazole, rabeprazole, timoprazole, picoprazole and tenatoprazole.

12. The oral pharmaceutical formulation according to claim 1, wherein said coating composition of said acetylsalicylic acid microcapsules represents 5 to 50% by weight, based on the total mass of the microcapsules.

13. The oral pharmaceutical formulation according to claim 1, wherein the diameter of said acetylsalicylic acid microcapsules is less than or equal to 1000 µm.

14. The oral pharmaceutical formulation of claim 1 for use in the treatment of chronic cyclooxygenase-2 mediated disease or condition i.e. inflammatory diseases, rheumatoid arthritis and systemic lupus erythematosis while decreasing the risk of thrombotic cardiovascular event.

15. The oral pharmaceutical formulation of claim 1 for use in the treatment of chronic cyclooxygenase-2 mediated disease or condition i.e. inflammatory diseases, rheumatoid arthritis and systemic lupus erythematosis, while decreasing the bleeding and/or ulceration of the stomach.

## Patentansprüche

1. Orale pharmazeutische Formulierung, enthaltend:
• mindestens einen Cyclooxygenase-2-selektiven Inhibitor aus der Gruppe bestehend aus Rofecoxib, Etoricoxib, Celecoxib, Valdecoxib, Parecoxib, COX-189, BMS347070, Tiracoxib, ABT963, CS502 und GW406381 und
• Acetylsalicylsäure, die zur Bildung von Mikrokapseln mit einer Beschichtungszusammensetzung überzogen ist,
wobei die Beschichtungszusammensetzung
- mindestens ein filmbildendes (Co)polymer, das in den Fluiden des Magen-Darm-Trakts unlöslich ist;
- mindestens ein (Co)polymer, das in den Fluiden des Magen-Darm-Trakts löslich ist; und
- mindestens einen Weichmacher
enthält und die Beschichtung nicht magensaftresistent ist und nicht auf Basis des pH-Werts zerfällt,
wobei die Menge an Acetylsalicylsäure in der Formulierung zwischen 75 und 310 mg liegt und die Mikrokapseln ein solches Freisetzungsprofil aufweisen, daß 70% der Acetylsalicylsäure in 0,05 M Kaliumdihydrogenphosphat/Natriumhydroxid-Puffermedium bei einem pH-Wert von 6,8 zwischen 2 und 20 Stunden freigesetzt werden, bestimmt gemäß den Angaben der European Pharmacopoeia, 4. Auflage, mit dem Titel "Dissolution test for solid dosage forms": Type-II-Dissolutest, durchgeführt unter SINK-Bedingungen bei 37°C mit einer Testdosis von 10 mg Wirkstoff und unter Rühren mit 100 rpm, wodurch die Formulierung bei Einnahme in einer einzigen Verabreichung eine kontrollierte Acetylsalicylsäure-Absorptionskinetik in vivo induziert, die sich über mindestens 24 Stunden erstreckt, wobei die Acetylsalicylsäure-Absorption kleiner oder gleich 10 Gew.-% der 0,4 Stunden nach der Einnahme resorbierten Fraktion der Dosis, kleiner oder gleich 50 Gew.-% der 3,9 Stunden nach der Einnahme resorbierten Fraktion der Dosis und kleiner oder gleich 90 Gew.-% der 23 Stunden nach der Einnahme resorbierten Fraktion der Dosis ist.

2. Orale pharmazeutische Formulierung nach Anspruch 1, wobei:
(i) das filmbildende (Co)polymer aus der Gruppe bestehend aus nicht wasserlöslichen Derivaten von Cellulose, Polvinylacetaten und Mischungen davon ausgewählt ist;
(ii) das lösliche (Co)polymer aus der Gruppe bestehend aus stickstoffhaltigen Copolymeren, wasserlöslichen Derivaten von Cellulose, Polyvinylalkoholen, Polyoxyethylenen und Mischungen davon ausgewählt ist und
(iii) der Weichmacher aus der Gruppe bestehend aus Cetylalkoholestern, Glycerin, Glycerinestern, Phthalaten, Citraten, Sebacaten, Adipaten, Azelaten, Benzoaten, Pflanzenölen, Fumaraten, Maleaten, Oxalaten, Succinaten, Butyraten, Triacetin, Malonaten, Ricinusöl und Mischungen davon ausgewählt ist.

3. Orale pharmazeutische Formulierung nach Anspruch 1, wobei:
(i) das filmbildende Polymer in einem Anteil von 10 bis 90 Gew.-% auf Trockenbasis, bezogen auf die Gesamtmasse der Beschichtungszusammensetzung, vorliegt;
(ii) das lösliche (Co)polymer in einem Anteil von 2 bis 25 Gew.-% auf Trockenbasis, bezogen auf die Gesamtmasse der Beschichtungszusammensetzung, vorliegt und
(iii) der Weichmacher in einem Anteil von 2 bis 20 Gew.-% auf Trockenbasis, bezogen auf die Gesamtmasse der Beschichtungszusammensetzung, vorliegt.

4. Orale pharmazeutische Formulierung nach Anspruch 1, wobei die Beschichtungszusammensetzung ferner
(iv) ein zweites filmbildendes (Co)polymer, das hydrophil und wasserunlöslich ist und Gruppen trägt, die in den Fluiden des Magen-Darm-Trakts ionisiert werden,
enthält.

5. Orale pharmazeutische Formulierung nach Anspruch 4, wobei es sich bei dem zweiten filmbildenden (Co)polymer um ein wasserunlösliches, geladenes Acrylderivat handelt.

6. Orale pharmazeutische Formulierung nach Anspruch 4, wobei das zweite filmbildende (Co)polymer in einem Anteil von 0 bis 90 Gew.-% auf Trockenbasis, bezogen auf die Gesamtmasse der Beschichtungszusammensetzung, vorliegt.

7. Orale pharmazeutische Formulierung nach Anspruch 1, wobei die Beschichtungszusammensetzung ferner
(v) mindestens ein Tensid und/oder Gleitmittel
enthält.

8. Orale pharmazeutische Formulierung nach Anspruch 7, wobei das mindestens eine Tensid aus der Gruppe bestehend aus anionischen Tensiden, nichtionischen Tensiden und Mischungen davon ausgewählt ist.

9. Orale pharmazeutische Formulierung nach Anspruch 7, wobei das Tensid und/oder Gleitmittel in einem Anteil von 2 bis 20 Gew.-% auf Trockenbasis, bezogen auf die Gesamtmasse der Beschichtungszusammensetzung, vorliegt.

10. Orale pharmazeutische Formulierung nach Anspruch 1, die ferner einen Protonenpumpeninhibitor enthält.

11. Orale pharmazeutische Formulierung nach Anspruch 10, wobei der Protonenpumpeninhibitor aus der Gruppe bestehend aus Esomeprazol, Leminoprazol, Omeprazol, Pantoprazol, Pariprazol, Rabeprazol, Timoprazol, Picoprazol und Tenatoprazol ausgewählt ist.

12. Orale pharmazeutische Formulierung nach Anspruch 1, wobei die Beschichtungszusammensetzung der Acetylsalicylsäure-Mikrokapseln 5 bis 50 Gew.-%, bezogen auf die Gesamtmasse der Mikrokapseln, ausmacht.

13. Orale pharmazeutische Formulierung nach Anspruch 1, wobei der Durchmesser der Acetylsalicylsäure-Mikrokapseln kleiner oder gleich 1000 µm ist.

14. Orale pharmazeutische Formulierung nach Anspruch 1 zur Verwendung bei der Behandlung von chronischen durch Cyclooxygenase-2 vermittelten Erkrankungen oder Leiden, d.h. entzündlichen Erkrankungen, rheumatoider Arthritis und systemischem Lupus erythematodes, bei gleichzeitiger Verringerung des Risikos eines thrombotischen kardiovaskulären Ereignisses.

15. Orale pharmazeutische Formulierung nach Anspruch 1 zur Verwendung bei der Behandlung von chronischen durch Cyclooxygenase-2 vermittelten Erkrankungen oder Leiden, d.h. entzündlichen Erkrankungen, rheumatoider Arthritis und systemischem Lupus erythematodes, bei gleichzeitiger Verringerung des Blutens und/oder der Ulzeration des Magens.

## Revendications

1. Formulation pharmaceutique orale comprenant :
- au moins un inhibiteur sélectif de la cyclooxygénase-2 choisi dans le groupe constitué par le rofécoxib, l'étoricoxib, le célécoxib, le valdécoxib, le parécoxib, COX-189, BMS347070, le tiracoxib, ABT963, CS502 et GW406381 et
- de l'acide acétylsalicylique qui est enrobé d'une composition d'enrobage pour former des microcapsules,
dans laquelle ladite composition d'enrobage comprend :
- au moins un (co)polymère filmogène qui est insoluble dans les fluides du tractus gastro-intestinal ;
- au moins un (co)polymère qui est soluble dans les fluides du tractus gastro-intestinal ; et
- au moins un plastifiant,
et ledit enrobage est non entérique et ne se désagrège pas en fonction du pH,
dans laquelle la quantité d'acide acétylsalicylique dans ladite formulation est comprise entre 75 et 310 mg et lesdites microcapsules ont un profil de libération tel que 70 % de l'acide acétylsalicylique dans du milieu tampon de dihydrogénophosphate de potassium 0,05 M/hydroxyde de sodium à un pH de 6,8 est libéré en entre 2 et 20 heures, déterminé selon les indications de la Pharmacopée Européenne, 4^{ème} édition, intitulées « Essai de dissolution des formes solides » : dissolutest de type II effectué en conditions SINK, à 37 °C, à une dose d'essai de 10 mg de principe actif et avec agitation de 100 tours/min,
dans laquelle, lorsqu'elle est ingérée sous forme d'une administration unique, la formulation induit une cinétique d'absorption contrôlée de l'acide acétylsalicylique in vivo, s'étendant sur au moins 24 heures, l'absorption de l'acide acétylsalicylique étant : inférieure ou égale à 10 % en poids de la fraction absorbée de la dose au bout de 0,4 heure après ingestion, inférieure ou égale à 50 % en poids de la fraction absorbée de la dose au bout de 3,9 heures après ingestion et inférieure ou égale à 90 % en poids de la fraction absorbée de la dose au bout de 23 heures après ingestion.

2. Formulation pharmaceutique orale selon la revendication 1, dans laquelle :
(i) ledit (co)polymère filmogène est choisi dans le groupe constitué par : les dérivés non hydrosolubles de la cellulose, les acétates de polyvinyle et les mélanges de ceux-ci ;
(ii) ledit (co)polymère soluble est choisi dans le groupe constitué par : les copolymères azotés, les dérivés hydrosolubles de la cellulose, les alcools polyvinyliques, les polyoxyéthylènes et les mélanges de ceux-ci ; et
(iii) ledit plastifiant est choisi dans le groupe constitué par : les esters de l'alcool cétylique, le glycérol, les esters du glycérol, les phtalates, les citrates, les sébacates, les adipates, les azélates, les benzoates, les huiles végétales, les fumarates, les malates, les oxalates, les succinates, les butyrates, le triacétate de glycéryle, les malonates, l'huile de ricin et les mélanges de ceux-ci.

3. Formulation pharmaceutique orale selon la revendication 1, dans laquelle :
(i) ledit polymère filmogène est présent en proportion de 10 à 90 %, en poids sur une base sèche par rapport à la masse totale de ladite composition d'enrobage ;
(ii) ledit (co)polymère soluble est présent en proportion de 2 à 25 %, en poids sur une base sèche par rapport à la masse totale de ladite composition d'enrobage ; et
(iii) ledit plastifiant est présent en proportion de 2 à 20 %, en poids sur une base sèche par rapport à la masse totale de ladite composition d'enrobage.

4. Formulation pharmaceutique orale selon la revendication 1, dans laquelle ladite composition d'enrobage comprend en outre :
(iv) un second (co)polymère filmogène qui est hydrophile et insoluble dans l'eau et qui porte des groupes qui sont ionisés dans les fluides du tractus gastro-intestinal.

5. Formulation pharmaceutique orale selon la revendication 4, dans laquelle ledit second (co)polymère filmogène est un dérivé acrylique chargé insoluble dans l'eau.

6. Formulation pharmaceutique orale selon la revendication 4, dans laquelle ledit second (co)polymère filmogène est présent en proportion de 0 à 90 %, en poids sur une base sèche par rapport à la masse totale de ladite composition d'enrobage.

7. Formulation pharmaceutique orale selon la revendication 1, dans laquelle ladite composition d'enrobage comprend en outre :
(v) au moins un tensioactif et/ou lubrifiant.

8. Formulation pharmaceutique orale selon la revendication 7, dans laquelle l'au moins un tensioactif est choisi dans le groupe constitué par : les tensioactifs anioniques, les tensioactifs non ioniques et les mélanges de ceux-ci.

9. Formulation pharmaceutique orale selon la revendication 7, dans laquelle le tensioactif et/ou lubrifiant est présent en proportion de 2 à 20 %, en poids sur une base sèche par rapport à la masse totale de ladite composition d'enrobage.

10. Formulation pharmaceutique orale selon la revendication 1, comprenant en outre un inhibiteur de la pompe à protons.

11. Formulation pharmaceutique orale selon la revendication 10, dans laquelle l'inhibiteur de la pompe à protons est choisi dans le groupe constitué par l'ésoméprazole, le leminoprazole, l'oméprazole, le pantoprazole, le pariprazole, le rabéprazole, le timoprazole, le picoprazole et le tenatoprazole.

12. Formulation pharmaceutique orale selon la revendication 1, dans laquelle ladite composition d'enrobage desdites microcapsules d'acide acétylsalicylique représente 5 à 50 % en poids, sur la base de la masse totale des microcapsules.

13. Formulation pharmaceutique orale selon la revendication 1, dans laquelle le diamètre desdites microcapsules d'acide acétylsalicylique est inférieur ou égal à 1000 µm.

14. Formulation pharmaceutique orale selon la revendication 1 destinée à être utilisée dans le traitement d'une maladie ou affection chronique à médiation par la cyclooxygénase-2 c'est-à-dire de maladies inflammatoires, de la polyarthrite rhumatoïde et du lupus érythémateux disséminé tout en diminuant le risque d'événement cardiovasculaire thrombotique.

15. Formulation pharmaceutique orale selon la revendication 1 destinée à être utilisée dans le traitement d'une maladie ou affection chronique à médiation par la cyclooxygénase-2 c'est-à-dire de maladies inflammatoires, de la polyarthrite rhumatoïde et du lupus érythémateux disséminé, tout en diminuant le saignement et/ou l'ulcération de l'estomac.
